# EUROPEAN PATENT APPLICATION

(11) **EP 1 374 892 A1**
(43) Date of publication of application: **02.01.2004**
(21) Application number: 02014397.0
(22) Date of filing: 28.06.2002
(51) Int. Cl.: A61K 39/095, A61P 31/04

(54) **Medicament for the treatment of diseases due to infection by Neisseria Meningitidis**

(71) Applicant: Braun, Jan Matthias, Dr., 50933 Köln (DE)
(72) Inventor: The designation of the inventor has not yet been filed
(74) Representative: Meyers, Hans-Wilhelm, Dr.Dipl.-Chem.

(57) **Abstract**

The subject of the invention is a medicament for the treatment of diseases due to infection by *Neisseria meningitidis*, which comprises glycoconjugates and/or lipooligosaccharides (LOS) from commensal bacteria with cross-reactive antigens to *Neisseria meningitidis* and/or antibodies against such glycoconjugates and/or lipooligosaccharides.

## Description

The subject of the invention is a medicament for the treatment of diseases due to infection by *Neisseria meningitidis,* which comprises glycoconjugates and/or lipooligosaccharides (LOS) from commensal bacteria with cross-reactive antigens to *Neisseria meningitidis* and/or antibodies against such glycoconjugates and/or lipooligosaccharides.

Disease due to *Neisseria meningitidis* (NM) can kill a previously healthy child or young adult within hours of the first symptoms of illness. Meningococcal disease is the largest single cause of childhood death in the developed world. Worldwide over 350,000 fatalities caused by NM were registered by the World Health Organisation (WHO) per annum.

Meningococcal disease can manifest itself in two main forms, meningitis and septicaemia. Meningococcal meningitis is an inflammation of the meninges, the membrane lining the brain and the spinal cord. In both, fulminant meningococcal septicaemia and meningococcal meningitis damage is caused by an uncontrolled localised or systemic host inflammatory response.

Meningococcal septicaemia, or blood poisoning, is caused by invasion of meningococci into the blood system of the patient. The host's immune defence is unable to kill and clear the invading pathogen successfully, or to neutralize meningococcal toxins. During the evasion of the immune response or due to treatment with antibiotics, meningococci shed endotoxin or lipooligosaccharide (LOS) into the blood system. In the absence of specific or cross-reactive neutralising antibodies, endotoxin induces a massive inflammatory response characterised by increased secretion of inflammatory mediators such as interleukin 1 (IL-1), interleukin-6 (IL-6), interleukin 8 (IL-8), tumour necrosis factor alpha (TNF α), interferon gamma (IFN γ) and acute phase proteins. Severity and fatality of the disease has been correlated with levels of inflammatory mediators detected in the blood.

In susceptible patients, this release of endotoxin and the results of inflammatory responses lead to rapid deterioration and failure of the normal homeostatic mechanisms. The removal of free endotoxin and the intervention in controlling the inflammatory response to endotoxin are crucial in preventing further damage to the host. Disseminated intravascular coagulation or blood platelet aggregation can result in the loss of limbs. Bleeding or leaking of peripheral blood into the surrounding tissue of blood vessels is recognised by the typical spots under the skin. The loss of perfusion may lead to the patient falling into a coma. Myocardial depression and multiple organ failure can lead to death.

Because meningococci are transmitted by aerosols or close ("kissing") contact, immunisation is the only effective means for prevention of disease in individuals lacking protective immunity.

NM is an exclusively human pathogen. It is Gram-negative, 1µm in diameter, aerobic diplococcus and shows a large degree of phenotypic variation.

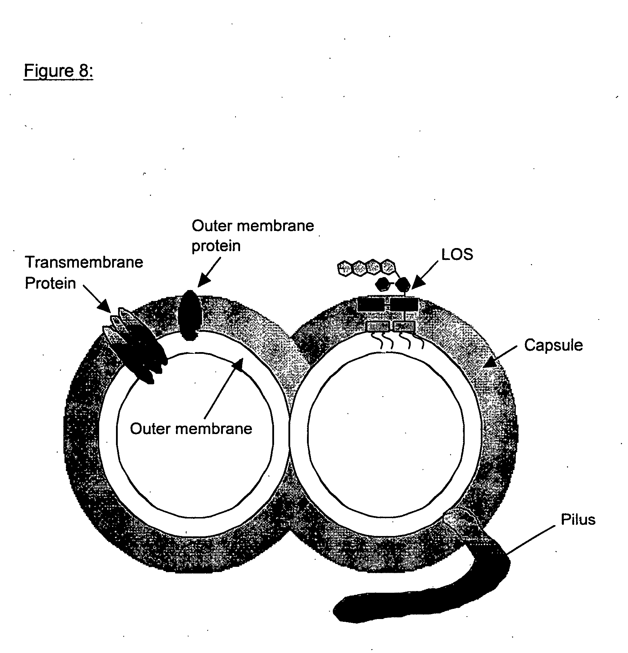

The major antigens of the outer membrane of meningococci vary greatly and these variations have been exploited to develop typing systems for epidemiological surveillance. These include capsular polysaccharide, a variety of outer membrane proteins, pili and endotoxin. The main antigens are found anchored to the typical Gram-negative envelope (Figure 8).

The first major success in development of vaccines against meningococcal disease was through the recognition of the immunogenicity of the capsular polysaccharide of these bacteria.

Originally meningococci were divided into serogroups for epidemiological purposes based on agglutination of capsular antigens. Currently 12 major antigenically distinguishable polysaccharide capsules have been identified: A, B, C, H, I, K, L, X, Y, Z, 29E and W135 which vary in their composition and arrangements of oligosaccharide units. The most prevalent serogroup structures are presented in table 1 The majority of meningococcal disease is caused by serogroup A, B and C. Groups B and C are responsible for most disease in Europe and the Americas while group A is more prevalent in Africa, Russia and causes periodic epidemics in Romania.

**Table 1:**

| Oligosaccharide structures of the major pathogenic meningococcal polysaccharide capsules | | |
|---|---|---|
| Sero-group | Capsular Polymer | |
| A | N-acetyl-3-0-acetyl mannosamine phosphate (α1→6), (*O*-acetylated-2-acetamido-2-deoxy-D-mannose-6-phosphate) | |
| B | Up to 200-residue polysaccharide units of (2→ 8) linked *N*-acetylneuramic acid | |
| C | O-acetylated or non acetylated (2→9) linked *N*-acetylneuramic acid | |
| X | *N*-acetyl glucosamine phosphate (α1→4), or ( 2-acetamido-2dedoxy-D-glucose-4-phosphate) | |
| Y | *N*-acetyl neuraminic acid:glucose, partially *O*-acetylated alternating sequences of D-glucose and *N*-acetylneuramic acid | |
| W-135 | 4-*O*-α-D-galactopyranosyl-β-D-N-acetyl-neuraminic acid, alternating sequences of D-galactose and N-acetylneuramic acid | |

Polysaccharide capsules are an effective way for pathogens to evade the human immune responses. Compared with non-capsulate meningococci, which are usually eliminated by bactericidal and opsonising antibodies in human serum, heavy polysaccharide capsulation is thought to reduce the ability of complement to bind and kill meningococci. Group B meningococci express a poorly immunogenic α2→8 linked poly-sialic capsule similar to some human antigens such as the Neural Cell Adhesion Molecule (N-CAM).

### Capsular antigen vaccines

Capsular polysaccharide vaccines against the serogroups A, C, Y, and W-135 induce protective immunity against these meningococcal serogroups in older children and adults. While these vaccines appear to be effective in adults, vaccines based on meningococcal polysaccharides are less effective in young children. Group C vaccines are thought to be ineffective in children younger than 2 years of age, and in children under 6 months for group A vaccines. The duration of protection elicited by capsular antigens is thought to be short lived, varying between two to four years after administration of the vaccine in adults and children.

This lack of wide-scale protection within the young age group (6 months to 5 years) that is most susceptible to meningococcal disease led to the development of conjugated group C vaccine. The principal is that used for the successful development of a vaccine for *H. influenzae* type b (Hib) in which the polysaccharide was conjugated to a carrier protein. Conjugation of polysaccharides to protein carriers induces a T-cell dependent response compared to polysaccharide alone which induces a T-cell independent response. Large molecular weight polysaccharide antigens like the meningococcal capsule bind to several receptors on B cells followed by crosslinking of these receptors. This triggers the production of immunoglobulin IgM and the transformation of the stimulated B cell into plasma cells. This T-cell independent immunity is short lived and does not generate memory.

The protein-carbohydrate antigen is ingested by antigen presenting cells (APC) and expressed on their cell surface within the major histocompatible complex type II receptor (MHC II). T-helper (T_{H}) cells expressing the MHC II receptor (CD4) and CD28 (B7 receptor) are activated by the APC leading to clonal proliferation and T_{H} cell maturation with some developing into memory T-cells. The activated T_{H} cells lead to differentiation of B-cells that bind directly to the hapten, *i.e.* a second encounter with the antigen or antigen present on the APC, followed by proliferation of B-cells, their differentiation into antibody producing plasma cells, or memory B-cells. This T-cell dependent immunity is long lasting and able to produce a wide range of classes of immunoglobulin. A serogroup C conjugate vaccine was introduced as part of a mass vaccination program in the United Kingdom in the autumn of 1999. Initial observations on the effectiveness of the conjugated vaccine in Scotland following mass vaccination of children and young adults indicate a reduction in disease caused by group C meningococci.

### The remaining problem of serogroup B

While the conjugate C vaccine appears to have partly reduced disease due to this serogroup, the NeuNAc capsule of group B meningococci is thought to be ineffective due its low immunogenicity and its presence on some human tissues *(i.e.,* neural cell adhesion molecule, N-CAM). Poly-sialylated N-CAM is an antigen found in several tumours associated with the immune evasion of some malignant metastatic cells. Protein vaccines containing the B capsular antigen did not show such effects in animal models or in humans. Vaccines based on group B capsular polysaccharide are poorly immunogenic, and short lived. It rarely induces antibodies in patients. Attempts to increase the immunogenicity by conjugation with protein carriers were not successful.

Because of the problems outlined above, other surface, antigens of meningococci have been assessed for their use as vaccine candidates for serogroup B meningococci.

While pili are associated with colonisation but not with invasive disease, the pilus antigens have not been assessed as vaccines because of the hypervariability of their terminal protein sequences.

*Neisseriae* species have a typical Gram-negative envelope consisting of a lipid bi-layer around a semi-rigid peptidoglycan sheet. Proteins can be anchored to the outer lipid layer alone, but usually form monomeric or polymeric structures penetrating both lipids and peptidoglycan layers (trans-membrane OMP).

Five classes of outer membrane proteins have been identified in NM. The classification is based on the molecular weight of proteins separated by sodium dodecyl sulphate (SDS)-polyacrylamide electrophoresis. Monoclonal antibodies to class 2 and class 3 outer membrane proteins have been used in epidemiological typing of meningococci (serotype), as have monoclonal antibodies to class 1 to determine the subtype. Class 1 OMP are porins selective for cations and are expressed on meningococcal isolates obtained from carriers and patients. There is antigenic variability within the class 1 OMP which has been used to develop monoclonal antibodies used in epidemiological surveillance. Investigations into the ability of OMP to elict antibodies showed that deglycosylation of all investigated classes (1-5) resulted in no significant antibody production *in vivo.* This suggests that antigenicity of OMP depends on post-translational glycosylation, or the presence of other oligosaccharides that must be considered in evaluation of these surface components for their use in vaccines.

In addition to the five major classes of outer membrane proteins, several other molecules are present on the meningococcal surface. Several iron binding OMP with variable molecular weights are currently under investigation as potential vaccine candidates for meningococci

### Outer membrane vesicle (OMV) vaccines

Various vaccines derived from deglycosylated OMV were tested in animal models and in clinical trials in Norway, Brazil, Cuba and Chile. These vaccines induced bactericidal antibodies in the immunised group but were protective mainly against strains expressing the serotype/subtype antigens of the strain from which the vaccine was produced. There was limited protection against strains expressing other OMP antigens. Due to the heterogeneity of antigens on meningococcal strains and the introduction of new strains into the vaccinated population, the use of OMV would only be effective in closed populations (*e.g.*, Cuba).

### Lipooligosaccharides (LOS)

All Gram-negative species have a family of glycolipids called endotoxin embedded into the hydrophobic outer membrane lipid layer. These macromolecules share a common basic structure consisting of :
a basal lipid A region anchored into the outer membrane;
a rough (R) core region consisting of a backbone of 2-keto-3-deoxyoctulosonic acid (KDO) and/or heptose (Hep) phosphate;
a highly variable region of saccharide domains differing in length and composition bound to the core heptose residue.

Lipid A is a phosphorylated di-glucosamine disaccharide substituted with fatty acids of variable length, and it is responsible for the biological activity which induces inflammation (endotoxin). Enteric Gram-negative species show a characteristic long linear chain of polysaccharide, called O-antigen, linked to the R-core giving the endotoxin of these species the name lipopolysaccharide (LPS). In contrast, the saccharide chains of all *Neisseria* species consist of very few residues, giving its endotoxin the name lipooligosaccharide (LOS).

De-glycolysated OMV vaccines showed poor immunogenicity after the removal of its toxic LOS moieties. LOS appears to be an essential component of anti-meningococcal protein vaccines, perhaps acting as an adjuvant in the human host.

### Variation in meningococcal LOS and immunotyping

Thirteen major LOS types were identified for *N. meningitidis* using polyclonal and monoclonal antibodies by passive haemagglutination inhibition techniques and whole cell ELISA. The majority of meningococcal isolates express one or more of the immunotypes L1-L12, while non-typable and L13 immunotypes are rare. The twelve major LOS types have a relative molecular weight ranging from 3.15 to 7.1 kDa. The oligosaccharide chain, also referred to as the α-chain or variable LOS region one (R1), is composed of the saccharides glucose (Glc), galactose (Gal), N-acetylglucosamine (GlcNAc), N-acetylgalactosamine (GalNAc). Sialylated forms contain the terminal saccharide N-acetylneuramic acid (NeuNAc) which is added to terminal galactose residues by endogenous or exogenous sialyl transferases. Abbreviations for core moieties are used as follows: glycero-D-manno-heptopyranoside (Hep or heptose); phosphoethanolamine (PEA); 2-keto-3-deoxyoctulosonic (KDO).

The complete structures of immunotypes L10 - L13 are not elucidated, but there is evidence that L10 contains the paragloboside residue and L11 shows some homology with L1. The PEA residue of immunotype L2 can be expressed in two forms that undergo phase variation: The PEA on the G3 region can be linked in (1→6) or (1→7) conformation; and the PEA can be replaced by a hydrogen (H) atom. The PEA residue of immunotypes L4 and L6 express both PEA (1→6) and (1→7) linkages. The expression of meningococcal immunotypes is associated with serogroups. Immunotypes L8, L9, L10, L11 and L12 are found on group A strains, while serogroup B and C meningococci express immunotypes L1 - L8.

### Immunotypes and pathogenicity

LOS immunotype expression is thought to be linked to the pathogenicity of the organism. Immunotypes L(3,7,9) are isolated predominantly from patients with invasive meningococcal disease. Other immunotypes are found predominantly among carrier strains. Immunotypes L3, L7 and L9 are thought to be similar in their immunochemical structures with immunotype L3 being sialylated by endogenous sialyl transferases. Immunotypes L3 and L7 are found on serogroup B and C meningococci and they have similar G2 core components, PEA (1→3) HepII. Immunotype L9 is expressed on group A strains.

The presence of the sialylated phenotype on invasive meningococci is associated with resistance to complement-mediated killing by masking the terminal galactose with NeuNAc. This mechanism is thought to reduce the recognition of the epitope by anti-LOS antibodies directed against the non-sialylated epitopes. Free or membrane bound sialyl-L(3,7,9) also upregulates neutrophil activation markers and results in increased injury of epithelial cell lines. Sialyl L(3,7,9) phenotypes can evade the complement mediated bacteriolysis cascade. This phenotype also reduces complement and anti-LOS antibody mediated phagocytosis by professional phagocytes.

### Expression of major and minor immunotypes by N. meningitidis

Meningococci are able to express more than one immunotype. Isolates from patients with meningococcal disease in the Netherlands (1989-1990) showed different immunotype combinations (Scholten R.J., Kuipers B., Valkenburg H.A., Dankert J., Zollinger W.D., and Poolman J.T. (1994), *J.Med.Microbiol.* **41**(4):236-43).
1. Group A meningococci L**9** (54%), L**9,8** (8%), L**10** (24%), L**10,11** (8%) and non-typable (**NT**) (8%).
2. Group B meningococci L**1** (1%), L**1,8** (11%), L**2** (10%), L**3,7** (36%), L**3**,**7**,**1** (4%), L**3**,**7**,**1**,**8** (2%), L**3**,**7**,**8** (28%), L**4** (4%), and L**8** (5%).
3. Group C meningococci L**1,8** (2%), L**2** (30%), L**3,7** (37%), L**3,7,1** (1%), L**3,7,1,8** (3%), L**3,7,8** (7%), L**4** (15%), L**8** (3%), and **NT** (3%).

The expression of multiple immunotypes within a meningococcal population allows the organism to diversify its antigenic structure. Selective pressure due to the presence of antibodies in the host to one LOS immunotype allows the strain to express other immunotypes increasing their chance of survival. This ability of meningococci to alter its LOS structure has to be taken into account in understanding the development of natural immunity, and in the choice of immunotypes as potential vaccine candidates. Sialylation and the expression of paragloboside gene cluster IgtABE are the main phase variable phenotypes known.

The expression of meningococcal immunotypes undergoes phase variation due to *in vitro* growth conditions. The variability of meningococcal phenotypes and LOS expression depends on the growth rate and phase, as well as the presence of exogenous sialyl transferases.

### Structural homology between LOS and human blood group antigens

Some LOS residues mimic human blood group antigens (Table 2).

**Table 2:**

| Homology of human blood group antigens with meningococcal LOS residues. | |
|---|---|
| oligo-saccharide: | α chain moiety: |
| P1 blood group | Galα (1→4) Galβ (1→4) GlcNAcβ (1→3) Galβ (1→4) Glcβ |
| p^{k}, CD77 | Galα (1→4) Galβ (1→4) Glcβ |
| P globoside | GalNAcβ (1→3) Galα (1→4) Galβ (1→4) Glcβ |
| Para-globoside | Galβ (1→4) GlcNAcβ (1→3) Galβ (1→4) Glcβ |
| i a determinant | Galβ (1→4) GlcNAcβ (1→3) Galβ (1→4) GlcNAcβ(1→3) Galβ (1→4) Glcβ |
| i b determinant | Sialyl-Galβ (1→4) GlcNAcβ (1→3) Galβ (1→4) GlcNAcβ(1→3) Galβ (1→4) Glcβ |
| Cer-dihexocide | Galβ (1→4) Glcβ |
| Gal, galactose; GlcNAc, N-acetylglucosamine; Glc, glucose; Sialyl, sialyc acid; cer, ceramide; the Glcβ on the reduced end is linked to (1→1) ceramide | |

The G1 regions of L1 and L11 LOS show identical terminal oligosaccharide residues of ceramide trihexocide, the p^{k} blood group antigen (CD77, globoside). The lacto-N-neotetranose of immunotypes L2, L(3,7,9) and L5 are identical to paragloboside, a precursor of P1 blood group antigen found in 75% of Caucasians, and the 3 terminal sugars are present in the Ii antigen. Immunotype L6 shares its two terminal sugars with the P blood group antigen, and L8 shares its terminal disaccharide with the common precursor of the P blood group system and steroid receptors. Both blood group antigens and meningococcal LOS with a terminal galactose residue can exist as sialylated and non-sialylated forms

### LOS vaccines

Meningococcal LOS vaccine induce a strong and potentially fatal inflammatory response due to the toxicity of its lipid A component. The oligosaccharide from which lipid A has been removed is not immunogenic. Meningococcal LOS is closely associated with the severity and fatality of disease. This is mainly due to its involvement in inducing large amounts of pro-inflammatory cytokines in a CD14 dependent mechanism. Anti-meningococcal LOS antibodies are not only bactericidal, but also opsonising in nature, resulting in the phagocytosis of invading bacteria and LOS containing blebs by human monocytes. Normal human serum of adults usually contains antibodies against meningococcal LOS, suggesting its important role in development of natural immunity to meningococcal disease.

The problem underlying the present invention is to provide new highly effective therapeutics and vaccines for diseases caused by *N. meningitidis.* A problem is especially to provide effective theurapeutics and vacchines agaist serogroup B, which are not available. The vacchines should be highly immunogenic, have a long-lasting effect and have low levels of toxicity, being therefore safe and effective in children and adults.

Surprisingly, the problem is solved by a medicament for the treatment or prevention of diseases due to infection by *Neisseria meningitidis,* which comprises glycoconjugates and/or lipooligosaccharides (LOS) from commensal bacteria with cross-reactive antigens to *Neisseria meningitidis* or antibodies against such glycoconjugates or lipooligosaccharides.

In a preferred embodiment of the invention, the medicament is a vaccine. The use of LOS from commensal bacteria as a vaccine has several advantages over other vaccines. The most common meningococcal LOS immunotype associated with disease is L(3,7,9) found in both group B and C outbreak strains of meningococci in Europe and America. The anti-meningococcal vaccine obtained from commensal bacteria which comprise glycoconjugates and/or LOS cross-reactive with meningococcal LOS including immunotype L(3,7,9) is effective against more than 90% of meningococcal outbreak strains worldwide. The LOS is highly immunogenic in all age groups, including young children, leading to long lasting protective immunity.

"Commensal" means species of *Neisseria* or closely related species, which are not *Neisseria meningitidis,* with a human host. The commensal bacteria with cross-reactive antigens to *Neisseria meningitidis* are preferably *Moraxella catarrhalis* (MC) or *Neisseria lactamica* (NL).

*N. lactamica* is a non-pathogenic commensal, rarely reported to cause disease in humans. The structures and expression of LOS of NL appear to be as diverse as those of meningococci. Kim *et al.* (Kim J.J., Mandrell R.E., and Griffiss J.M. (1989) *Infect.Immun.* **57**(2): p. 602-608) identified epitopes common to NL which were recognised by two monoclonal antibodies produced against NM. The antibody D6A bound to meningococcal immunotypes L1, L8, L(3,7,9), L10 and L11. The antibody 06B4 bound to L2, L4, L8 and L(3,7,9) immunotypes.

None of these immunotypes share any of the P-blood group related saccharides of the G1 terminal α-chain of meningococcal LOS. Later analysis of the LOS structure revealed that these immunotypes share a common core structure, the G2 and G3 region of the second core heptose (HepII). The third carbon shares a hydroxyl (-OH) group, and the fourth carbon contains a PEA residue.

*M. catarrhalis* (MC) is a commensal Gram-negative diplococcus previously classified within the genera *Branhamella* and *Neisseria.* Recent genetic studies resulted in the re-classification of MC into the genus *Moraxella.*

Although MC is associated with some childhood diseases, it is frequently isolated as a commensal from the respiratory tract in healthy young children. Children are colonised with 3-4 different strains of MC within the first two years of life. It is isolated more frequently than *Neisseria* species during the first 6 months of life when infants are developing antibodies to the bacteria in their environment. One member of the genus, *Moraxella nonliquefaciens* expresses a capsular antigen similar to group B meningococci and *E*. *coli* K1, and it is isolated from about 20% of healthy carriers.

Several surface antigens are thought to be involved in the development of immunity to MC. Carriage and infections with MC are associated with the development of protective IgG from an early age. These include protein antigens and glycoconjugates. Two OMP are associated with protective immunity, UspA1 and UspA2, possibly due to structural homology and cross-reactivity detected with monoclonal antibodies.

Although LOS from MC differs structurally from meningococcal LOS, both species share some homology in their oligosaccharide chain moieties. Terminal oligosaccharide residues found on the non-reducing end of MC LOS share some homology with human blood group antigens. Combination of five different saccharide residues of the α or β chains determine the immunotype of MC LOS (Gal, galactose; Glc, glucose; GlcNAc, N-acetylglucosamine; KDO, 2-keto-3-deoxyoctulosonic):
1. Galα (1→4) Galβ (1→4) GlcNAcα (1→2) Glcβ;
2. Galα (1→4) Galβ (1→4) Glcα (1→2) Glcβ;
3. GlcNAcα (1→2) Glcβ;
4. Glcα (1→2) Glcβ;
5. Glcβ.

Preferably, the medicament of the invention is used for the treatment or vaccination for diseases caused by *Neisseria meningitidis* of the serogroup A, B, C or W135. The use against serotype B is of high significance, because the LOS of the commensal species, especially NL and MC, are of low toxicity in contrary to those of NM. Therefore, the medicament and vacchine of the invention allow for the first time an effective treatment and vacchination of diseases caused by serogroup B NM.

The glycoconjugates or lipooligosaccharides may be included in outer membrane vesicles, blebs, lipid layers, liposomes or killed or viable bacteria commensal to *Neisseria meningitidis.* Preferred Glycoconjugates used according to the invention are lipooligosaccharides and glycolipids. It is also advantageous to apply glycoconjugates, which are glycoproteins. Preferrably, immunogenic sugar moieties are conjugated to protein carriers.

In a preferred embodiment of the invention, the glycoconjugates or lipooligosaacharides are chemically modified, conjugated or hydrolized, preferably by mild acid hydrolysis. Preferably, mild acid hydrolysis is applied under conditions such that fragments of lipooligosaccharides are obtained.

The lipid A moieties, core, and oligosaccharide antigens that can be obtained through chemical modification of LOS from NL and/or MC induce cross-reactive, bactericidal, opsonophagocytic and anti-inflammatory (functional) antibodies. Further, genetic modification of the genes encoding the lipid A, core and oligosaccharide expression and assembly can be used to produce a native and/or structurally defined LOS. The use of glycosyltransferases associated with LOS synthesis and assembly onto a protein carrier, or (detoxified) lipid A, or liposome carrier allows the production of a synthetic molecule able to mimic and/or induce cross-reactive functional antibodies to be used as a vaccine and/or a medicament for the treatment of meningococcal disease.

Hydrolysis may for instance be performed in a way that immune-accessible glyco- or lipid A epitopes are obtained.

The antibodies which are part of the medicament of the invention may be monoclonal or polyclonal and of animal or human origin. Advantageously, they are obtained
from virus immortalized human lymphocytes secreting the glycoconjugate neutralizing, specific or cross-reactive antibodies,
from human lymphocytes secreting the neutralizing antibodies fused with a human hybridoma cell line, or
from immunized animals, preferably mice, rats, rabbits or pigs, producing polyclonal serum containing such antibodies, or
from immunized animals, preferably mice, rats, rabbits or pigs, after fusion of the animal lymphocytes with a human or animal hybridoma cell line.

If the medicament of the invention comprises such antibodies, it is preferably used for the treatment of acute meningitis or septicaemia as an anti-inflammatory, bactericidal and/or opsonophagocytosis inducing medicament.

The medicament of the invention is advantageously applied as a nasal or oral spray, as a liquid for injection, as an orally applied capsule or as a tablet. It may be applied in combination with an adjuvant.

A subject of the invention is also a diagnostic to assess the susceptibility of patients for diseases due to *Neisseria meningitidis,* which comprises glycoconjugates and/or lipooligosaccharides from from commensal bacteria with cross-reactive antigens to *Neisseria meningitidis* or antibodies against such glycoconjugates or lipooligosaccharides.

### Examples

### Bacterial strains

Standard immunotype strains of meningococci L1-L12 were obtained from Dr. W. D. Zollinger, Washington D.C. NL isolates were obtained from our culture collection. None of the isolates were agglutinated by standard serogroup reagents and none reacted with the monoclonal antibodies used to determine serotype or subtype of meningococci.

Cultures were grown overnight at 37°C on human blood agar (HBA) containing: lysed whole blood (100 ml) from the Scottish National Blood Transfusion Service (SNBTS); special peptone (23 g) (Difco); corn starch (1g) (Sigma); NaCl (4.5 g) (Sigma); D-glucose (1 g) (Sigma); technical grade agar (10 g) (OXOID); K₂HPO₄ (4 g) and KH₂PO₄ (1 g); 900 ml of distilled water.

### Bactericidal Assay

The microtiter plate method described by Zorgani *et al.* (Zorgani A.A., James V.S., Stewart J., Blackwell C.C., Elton R.A., and Weir D.M. (1996), *FEMS Immunol Med Microbiol.* **14**(2-3): p.73-81) was used to screen for bactericidal activity.

A pool was prepared with serum from eight healthy adult donors with no known history of meningococcal disease. The pool was inactivated at 56°C for 30 min, divided into aliquots which were absorbed twice at 4°C overnight with viable individual strains of NL (10¹⁰ bacteria ml⁻¹), centrifuged at 1000 × *g* and filter sterilized using a 0.22 µm membrane (Nu-flow, Oxoid). Aliquots of the absorbed sera were tested for sterility and stored at -70°C.

The complement source was prepared from a blood sample from a healthy adult volunteer with no known history of meningococcal disease. Serum was supplemented with 1mM EDTA to ensure the bactericidal activity observed reflected the classical antibody-mediated complement killing and not the alternative pathway. The serum was absorbed twice with a pool of the meningococcal test strains grown overnight at 37°C in a humidified atmosphere with 5% (v/v) CO₂ on HBA. The absorbed serum was sterilized through a 0.22 µm membrane filter. The complement source was tested for sterility and stored in aliquots at -70°C. Complement titres were assessed with sensitised sheep red blood cells and used in the assays at a dilution of 1 in 16. For testing in the bactericidal assay, strains were grown overnight on HBA and washed twice in PBS by centrifugation at 1000 × *g*. The total count for each strain was determined microscopically with a Thoma counting chamber and adjusted to approximately 10⁵ colony forming units (cfu) per ml in sterile PBS containing MgCl₂ (0.5 mM), CaCl₂ (0.9 mM) and glucose (0.1%, w/v) (Sigma) (pH 7.2).

Triplicate samples containing equal volumes (40ul) of the test strain (approximately 400 cfu/well) and the heat inactivated serum pool were incubated with 20 µl of the complement source for 30 min in sterile U-bottomed 96 well microtitre plates. Three drops (10 µl) from each sample well were placed on HBA plates which had been dried for 48 hours at room temperature. The plates were incubated overnight at 37°C, the mean cfu recorded and used to calculate the serum bactericidal activity. Each assay included two controls: 1) bacteria + complement source + D-PBS but no serum; 2) bacteria + heat inactivated complement source + absorbed or unabsorbed heat inactivated serum pool.

The absorbed and unabsorbed pools were tested in parallel and the bactericidal activity of the absorbed and unabsorbed pools were compared. Compared with results obtained with the unabsorbed serum, reduction in bactericidal killing ≥ 80% with the absorbed serum was taken as evidence that the NL strain had removed significant levels of bactericidal activity.

### Assay for inflammatory responses

The human monocytic cell line THP-1 was obtained from the European Collection of Animal Cell Cultures (ECACC, UK). Cells were grown to 10⁴ to 10⁶ cells ml⁻¹ in RPMI-1640 cell culture medium (Sigma, Poole, Dorset, UK) supplemented with foetal calf serum (FCS) (5%, v/v) (Gibco), L-glutamine (1%, w/v) (Gibco), penicillin (100 IU ml⁻¹) and streptomycin (200 mg ml⁻¹) (Gibco) for not more than 18 weeks after establishing the cell line. The calf serum did not contain antibodies against any of the bacterial strains tested as determined by whole cell ELISA (Scholten R.J., Kuipers B., Valkenburg H.A., Dankert J., Zollinger W.D., and Poolman J.T. (1994), *J.Med.Microbiol.* **41**(4):236-43).

The mouse fibroblast cell line L929 was obtained from the ECACC. Cells were grown in 75 cm³ tissue culture flasks (Greiner) to 70 % confluence in growth medium containing DMEM medium (Sigma) supplemented with FCS (5%, v/v) (Gibco), L-glutamine (1%, w/v) (Gibco), penicillin (100 IU ml⁻¹) and streptomycin (200 mg ml⁻¹) (Gibco) at 37 °C with 5% CO₂ (Gordon A.E., Al Madani O., Weir D.M., Busuttil A., and Blackwell C.C. (1999), *FEMS Immunol Med Microbiol.* **25**(1-2): p. 199-206).

### Extraction of LOS

All strains were grown for 18 h in 5% (v/v) CO₂ on HBA. Cells were harvested from plates, washed in sterile pyrogen free PBS, centrifuged at 1000 × *g* and resuspended in pyrogen free distilled water. The hot phenol-water method described by Hancock and Poxton (1988, Modern Microbiological Methods: Bacterial Cell Surface techniques John Wiley & Sons, Chichester, UK) was used to extract the LOS. The purified LOS contained protein contaminants of <1% (w/w) as assessed against a standard of bovine serum albumin (BSA) (Sigma). LOS was resuspended in RPMI-1640 medium (Sigma) and sterilized through a 0.22 µm membrane filter. Aliquots were stored at -70° C and two samples from each batch were incubated at 37° C for 18 h to test for sterility.

### Induction of pro-inflammatory cytokines

THP-1 cells were incubated for 72 h with 10⁻⁷ M VD3 to induce expression of the CD14 cell surface antigen (James S.Y., Williams M.A., Kelsey S.M., Newland A.C., and Colston K.W., 1997, *Biochem. Pharm.* **54**: p. 625-637). Triplicate samples of the differentiated or undifferentiated cells were challenged for 6 h with tenfold dilutions of LOS from the individual *Neisseria* strains or *Escherichia coli* endotoxin (strain 026: B6) (Sigma). A range of concentrations from 1 pg ml⁻¹ to 100 ng ml⁻¹ were examined in initial studies with LOS of the meningococcal immunotype strain L3. A concentration of 100 pg ml⁻¹ was used to assess the neutralising effects of pooled human serum or immune mouse serum. To determine the concentration to be used in the neutralising assays, human serum or immune mouse serum was serially diluted and tested using WCE to assess binding of IgG to cells of NL1. A final dilution of 1 in 1,000 was used in the neutralization experiments (Braun J.M., Blackwell C.C., Poxton I.R., El Ahmer O., Gordon A.E., Madani O.M., Weir D.M., Giersen S., and Beuth J., 2002, *J.Infect.Dis.* **185**:p. 1431-1438).

### Detection of cytokines

The ELISA to detect IL-6 and bioassay to detect TNFa reported previously were used in these studies (Braun J.M., Blackwell C.C., Poxton I.R., El Ahmer O., Gordon A.E., Madani O.M., Weir D.M., Giersen S., and Beuth J., 2002, *J.Infect.Dis.* **185**:p. 1431-1438).

### Immune mouse sera

Strain NL1 was grown on HBA and killed by heating for 60 min at 100°C. The bacteria (10⁹ in 100 µl) were injected in adjuvant free and pyrogen free saline (SIGMA) into the tail vene of three six week old male BALB/c mice on three consecutive days. This was followed by repeated intravenously inoculations with the same dose and batch of antigen at weeks 4, 8, 12 and 16. In week 20, LOS (100 µl, 100 ng ml⁻¹) obtained by hot phenol water extraction of NL strain was injected. Three days after the final injection, blood was collected aseptically by cardiac puncture, allowed to clot, centrifuged at 500 × *g* for 15 min at 4°C. The supernatant was collected and diluted in pyrogen free saline (1 in 100). Complement was inactivated by heat treatment (56°C for 30 min) and the sera were stored in aliquots (1 ml) at -70°C. The production of antibodies was covered by an animal licence obtained from the British Home Office.

Antibodies to NL1 in samples taken from the mice before immunisation and at the end of the immunisation schedule were detected by WCE.

### Statistical analysis

The mean, standard deviation (SD) and Student's t-test were calculated using Minitab for the Apple Mackintosh. To determine if the data were normally distributed, normal probability plots were used (Gardiner W.P., 1997, Statistics for the Biosciences. Prentice Hall Europe, Hertfordshire). Regression and analysis of variance showed that cytokine levels were normally distributed. Probability values were calculated with a confidence interval of 5% against the negative control treated with PBS only or the *E.coli* 026:B6 LPS. Two-sided analysis (5% confidence level) was carried out using a paired t-test for different endotoxin samples.

### Results

In three independent experiments, the absorbed and unabsorbed pools were tested for bactericidal activity against 7 isolates of NL from the following countries: Scotland (2); Iceland (1); the Czech Republic (1); and Greece (3) (Table 4). The results obtained were consistent in each of the experiments. Eighteen meningococcal isolates, including the twelve immunotype reference strains, were also tested in the bactericidal assays (Table 5). The unabsorbed pool killed all the strains tested; the cfu of each strain was reduced by ≥ 80% that of their respective controls.

### NL1 (Scotland)

Bactericidal activity against the following NL strains was absorbed by NL1: NL2 from Scotland; NL3 from Iceland; NL4 and NL5 from Greece; NL7 from the Czech Republic. One strain from Greece (NL6) was killed by the absorbed sera. Bactericidal activity against the following meningococcal strains was absorbed by *N. lactamica1:* immunotype reference strains C:NT:P1.2:**L1,8,** B:2a:P1.5,1.2:**L3,** C:11:P1.16:**L4**, B:4;P1.NT:**L5**, B:9:P1.1:**L7**, B:8,19:P1.7:**L8**, and A:21:P1.10:**L9**; B:15:P1.16 from England; B:15:P1.16 from Iceland; B:NT:NT, B:15:NT, from Scotland; B:2a:P1.2 from Greece.

### NL7 (Czech Republic)

Bactericidal activity against the following NL strains was absorbed by NL7: NL1 from Scotland; NL3 from Iceland. Bactericidal activity against the following meningococcal strains was absorbed by NL7: immunotype reference strains C:NT:P1.2:**L1**,8, C:2c:P1.1:**L2**, B:5:1.7,1:**L6**, B:9:P1.1:**L7**, and B:8,19:P1.7:**L8**; B:15:NT and B:NT:NT from Scotland; and NG:4:NT from Greece. All other strains were killed by the absorbed serum pool.

### NL3 (Iceland)

Bactericidal activity against the following NL strains was absorbed by NL3: NL1 and NL2 from Scotland; NL7 from the Czech Republic. Bactericidal activity against the following meningococcal strains was absorbed by NL3: immunotype reference strains C:2c:P1.1:**L2**, B:5:P1.7,1:**L6**, and B:9:P1.1:**L7**; B:15:NT and B:NT:NT from Scotland; NG:4:NT from Greece.

### NL6 (Greece)

Bactericidal activity against NL4, one of the other Greek isolates, was absorbed by NL6. Bactericidal activity against the following meningococcal isolates was absorbed by NL6: immunotype reference strain B9:P1.1:**L7**; and the Greek carrier isolate NG:4:NT. All other strains were killed by the unabsorbed and the absorbed serum pools.

**Table 3:**

| *N. meningitidis* immunotypes reference strains | | | |
|---|---|---|---|
| Strain | Code | Major LOS | Minor LOS |
| C:NT:P1.2 | 126E | L1 | 8 |
| C:2c:P1.1 | 35E | L2 | 3,7,9 |
| B:2a:P1.5, 2 | 6275 | L3 | 8 |
| C:11:P1.16 | 891 | L4 | |
| B:4:P1.NT | M981 | L5 | 3,7,9 |
| B:5:1.7, 1 | M992 | L6 | |
| B:9:P1.7, 1 | 6155 | L7 | 3, 8 |
| B:8:P1.7, 1 | M978 | L8 | 3, 4,7 |
| A 21:P1.10 | 120M | L9 | 6, 8 |

**Table 4:**

| Absorption of bactericidal antibodies of adult human pooled serum by *N, lactamica* | | | | | | |
|---|---|---|---|---|---|---|
| Phenotype | Origin | | NL1 | NL3 | NL6 | NL7 |
| NG:NT:NT | Scotland | *N. lactamica* 1 | + | + | - | + |
| NG:NT:NT | Scotland | *N. lactamica* 2 | + | + | - | - |
| NG:NT:NT | Iceland | *N. lactamica* 3 | + | + | - | + |
| NG:NT:NT | Greece | *N. lactamica* 4 | + | - | + | - |
| NG:NT:NT | Greece | *N. lactamica* 5 | + | - | - | - |
| NG:NT:NT | Greece | *N. lactamica* 6 | - | - | + | - |
| NG:NT:NT | Czech Republic | *N. lactamica* 7 | + | + | - | + |

**Table 5:**

| Absorption of bactericidal activity against meningococcal strains by *N*. *lactamica* isolates from different parts of Europe | | | | | |
|---|---|---|---|---|---|
| Phenotype | Origin | NL1 | NL3 | NL6 | NL7 |
| B:15:P1.7,16 | England | + | - | - | - |
| C:2a:P1.2 | Greece | + | - | - | - |
| NG:4:NT | Greece | - | + | + | + |
| B 15:P1.7,16 | Iceland | + | - | - | - |
| B:15:NT | Scotland | + | + | - | + |
| B:NT:NT | Scotland | + | + | - | + |
| C NT:P1.2: L1,8 | USA | + | - | - | + |
| C:2c:P1.1: L2 | USA | - | + | - | + |
| B:2a:P1.5,1.2:L3 | USA | + | - | - | - |
| C:11:P1.16:L4 | USA | + | - | - | - |
| B 4:P1.NT:L5 | USA | + | - | - | - |
| B:5:1.7,1:L6 | USA | - | + | - | + |
| B:9:P1.1:L7 | USA | + | + | + | + |
| B:8,19:P1.7:L8 | USA | + | - | - | + |
| A 21:P1.10:L9 | USA | + | - | - | - |

### Inflammatory responses to LOS of N. lactamica

Because NL1 absorbed bactericidal activity against the broadest range of NL and meningococcal strains, it was used in the following experiments to assess induction of inflammatory responses and neutralising activities of immune serum raised against the strain.

Initial experiments found that maximum levels of TNF and IL-6 were induced at 6 hours following exposure of the THP-1 cells to 100 pg ml⁻¹ of the LOS preparation from the meningococcal immunotype strain L3.

### TNFα responses to LOS of different species in the presence and absence of VD3

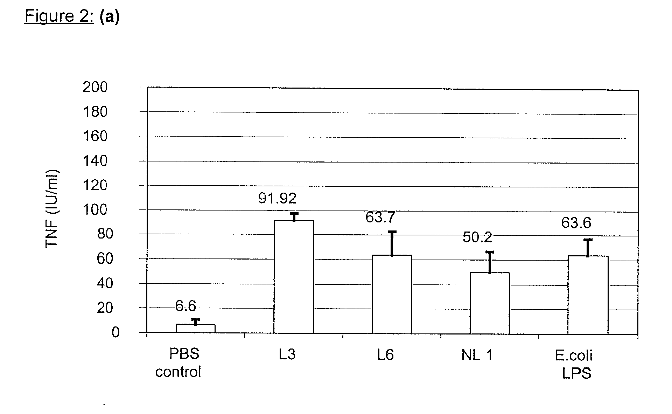

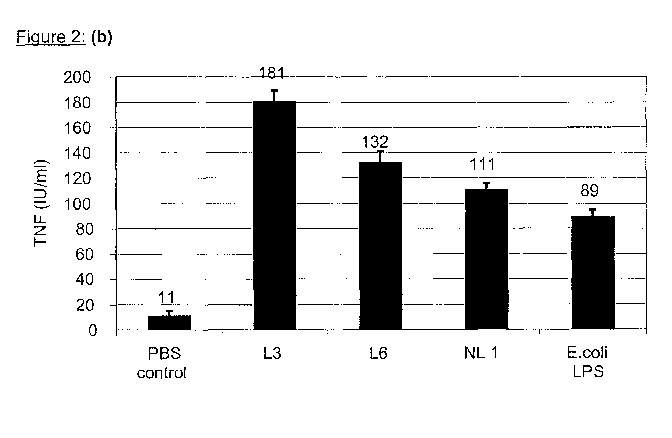

In 6 independent experiments, incubation of undifferentiated THP-1 cells with LOS (100 pg ml⁻¹) from immunotypes L3, L6, NL1 or *E. coli* endotoxin resulted in detection of low levels (50-92 IU ml⁻¹) of TNFα compared with cells incubated with PBS (Figure 2a).

Compared with levels obtained with the undifferentiated THP-1 cells, there was a significant increase in TNFα activity (p<0.01) for each of the LOS preparations with the VD3-differentiated cells (Figure 2b). All LOS samples showed significantly higher TNFα activity compared with the *E.coli* endotoxin. With the VD3 differentiated cells, the highest TNFα levels were obtained with LOS from the L3 immunotype. TNFα levels for NL1 and *E.coli* were significantly lower than those elicited by LOS meningococcal immunotype strains L3 or L6.

### IL-6 responses to LOS of different species in the presence and absence of VD3

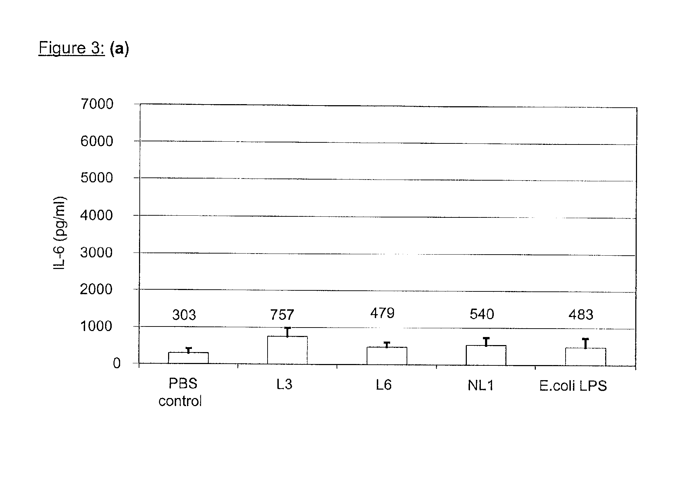

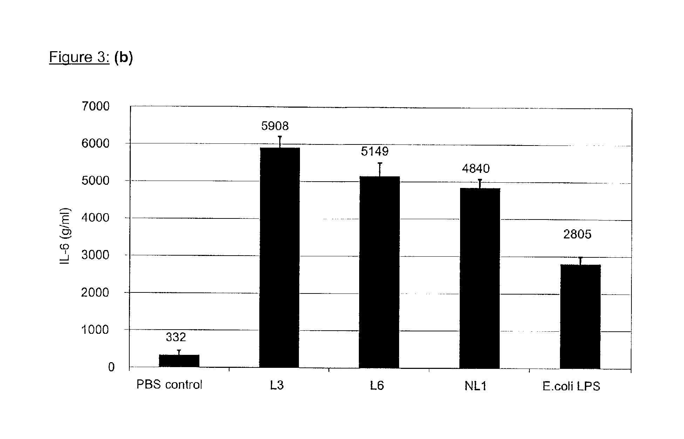

A similar pattern was observed for induction of IL-6. Compared with cells incubated with PBS, incubation of undifferentiated THP-1 cells with LOS from the different strains resulted in low levels of IL-6 production (Figure 3a). Compared with IL-6 levels obtained with the undifferentiated THP-1 cells, there was a significant increase in IL-6 levels for each of the LOS preparations for the differentiated cells (p<0.01) (Figure 3b). All meningococcal LOS preparations induced significantly higher levels of IL-6 compared with the *E. coli* endotoxin (Figures 3b). NL1 LOS and *E. coli* LPS elicited IL-6 levels significantly lower than those obtained with LOS from the L3 (P <0.01).

### Cytokine levels following treatment of LOS with pooled human serum

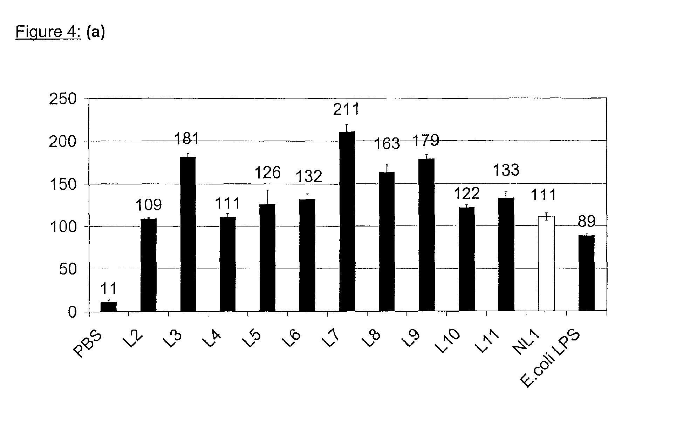

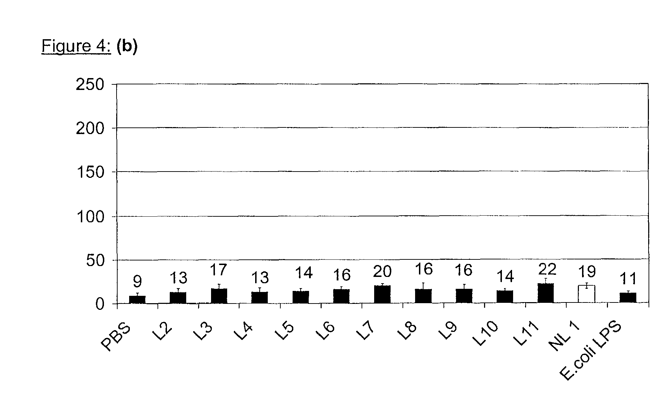

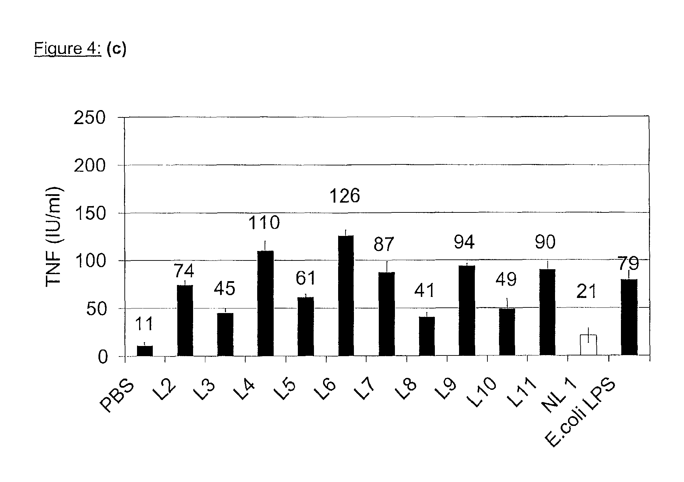

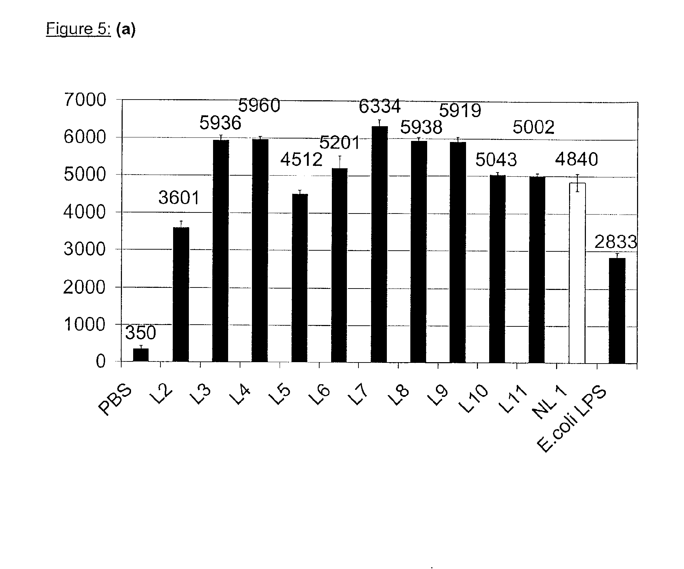

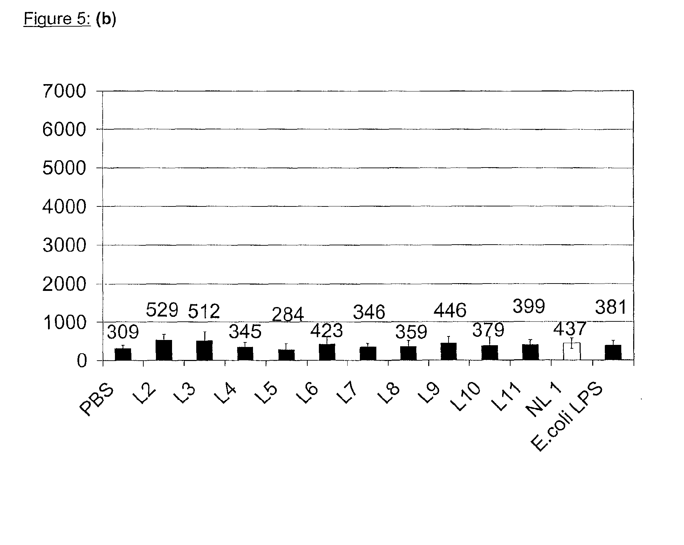

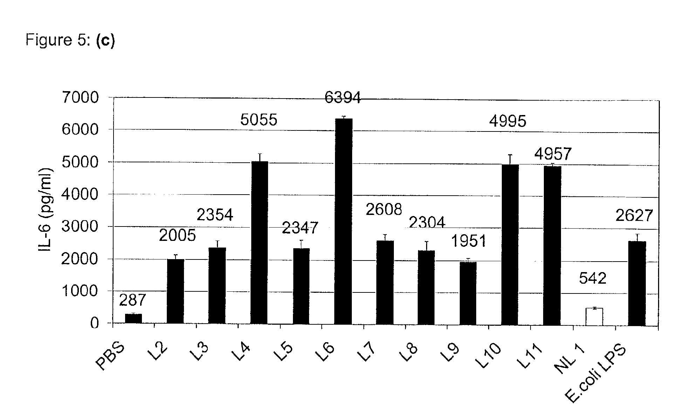

The pooled human serum from the bactericidal assays was incubated at a dilution of 1 in 1000 with LOS from the meningococcal immunotypes and LOS from NL1. The serum pool significantly reduced TNF and IL-6 responses elicited by each of the LOS preparations tested. (Figures 4b and 5b)

### Cytokine levels following treatment of LOS with immune mouse serum induced by the NL1 strain

In six experiments, TNFα and IL-6 levels for LOS from the following meningococcal immunotypes co-incubated with immune serum of mice vaccinated with strain NL1 were lower compared with cytokine levels obtained with LOS in the absence of serum: L2 (P<0.01); L3 (P<0.01); L7 (P<0.01); L8 (P<0.01); L9 (P<0.01); L11 (P<0.01). TNFα levels were lower for immunotypes L5 (P<0.01) and L10 (P<0.01), but IL-6 levels for these immunotypes were not significantly reduced (P>0.13). IL-6 levels were significantly lower for immunotype L6 (P<0.01), but TNFα levels were not significantly reduced (P=0.34). Cytokine levels for immunotype L4 were not significantly lower (P=0.27). Treatment of *E*. *coli* LPS with the immune mouse serum induced by NL1 reduced TNFα and IL-6 levels by not more than 15% (Figures 4c and 5c). The non-immune serum did not reduce cytokine levels in any of the endotoxin samples (P=0.89).

### Detection of blood group or immunotype antigens on NL from different sources

The binding of blood group antigens and LOS immunotypes to NL isolates from the Czech Republic, from Russian immigrant children in Greece, from an Icelanding NL strain, and isolates from Scotland were assessed by WCE. The binding of blood group antibodies (Table 6) and meningococcal immunotype antibodies are summarised by country (Table 7) and by region of Greek NL isolates (Table 8).

**Table 6:**

| Binding of antibodies to human blood group antigens by *N. lactamica* isolates from different European countries | | | | |
|---|---|---|---|---|
| | Scotland n=12 No (%) | Iceland n=1 No (%) | Russian children n=27 No (%) | Czech Rep. n=4 No (%) |
| P | 1 (8.3) | 1 (100) | 10 (37) | 1 (25) |
| P1 | 2 (16.7) | 1 (100) | 4 (14.8) | 2 (50) |
| p^{K} | 8 (66.7) | 0 (0) | 8 (29.6) | 2 (50) |
| paragloboside | 8 (66.6) | 1 (100) | 1 (3.7) | 4 (100) |
| I | 7 (58.3) | 1 (100) | 8 (29.6) | 4 (100) |
| n.t., not tested) | | | | |

**Table 7:**

| Binding of immunotyping antibodies by *N. lactamica* isolates from different European regions | | | | | |
|---|---|---|---|---|---|
| | Scotland n=12 No (%) | Iceland n=1 No (%) | Russian children n=27 No (%) | Greek children n=73 No (%) | Czech Rep. n=4 No (%) |
| L1 | 1 (8.3) | 0 (0) | 4 (14.8) | 24 (32.9) | 1 (25) |
| L(3,7,9) | 9 (75) | 1 (100) | 9 (33.3) | 58 (79.5) | 4 (100) |
| L8 | 2 (16.7) | 0 (0) | 4 (14.8) | 7 (9.6) | 2 (50) |
| L10 | 4 (33.3) | 0 (0) | 1 (3.7) | n.t. | 1 (25) |
| n.t., not tested | | | | | |

**Table 8:**

| Binding of monoclonal antibodies to meningococcal immunotypes by *N. lactamica* strains isolated from different regions in Greece and isolates from Russian immigrant children | | | | | |
|---|---|---|---|---|---|
| | FL | SR | SF | Greek children | Greece Russia |
| N | 28 | 28 | 17 | 73 | 27 |
| L1 | 7 (25) | 10 (35.7) | 7 (41.2) | 24 (32.9) | 4 (14.8) |
| L(3,7,9) | 23 (82.1) | 20 (71.4) | 15 (88.2) | 58 (79.5) | 9 (33.3) |
| L8 | 3 (10.7) | 2 (7.1) | 2 (11.8) | 7 (9.6) | 4 (14.8) |
| Cz, Czech Republic; GRE Athens, Greece; ICE, Iceland; SCO, Scotland; SR, Serres; SF, Euros; SF, Florina; MC, *M*. *catarrhalis;* NL, *N. lactamica* | | | | | |

There was no significant difference in the number of isolates from the Czech Republic (n=4) and Scotland (n=12) expressing blood group antigens of the P-or Ii-system. Significantly fewer isolates from Russian immigrant children in Greece (n=27) expressed pK (P=0.039), paragloboside (P<0.001), and Ii (P=0.042) blood group antiges.

There was no significant differences in the distribution of LOS immunotype cross-reactivity between NL samples from the Czech Republic, native Greek children, and isolates from Scotland, except that significantly more isolates obtained from native Greek children bound antibodies to immunotype L1 compared to isolates obtained from other regions in Europe (Kruskal-Wallis analysis of variance by ranks). Meningococcal immunotypes L(3,7,9) (P<0.02) and L10 (P<0.01) was expressed by fewer NL isolates obtained from Russian immigrant children in Greece compared to isolates obtained from either Scotland, the Czech Republic, or from native Greek children. Meningococcal immunotypes L10 (P<0.01) was expressed by fewer NL isolates from Russian immigrant children in Greece compared to samples from either Scotland or the Czech Republic (Table 7). There were no significant differences in the immunotype phenotypes from NL isolates isolated from native Greek children in the regions of Serres, Euros, or Florina (Table 8).

### Binding of cross-reactive antibodies obtained from BALB/c mice immunised with whole bacteria cells, LOS and/or OMV obtained from NL isolates

The development of cross-reactive antibodies via intravenous (i.v.) administration of whole cells (10⁹bacteria), LOS (50 ng) or OMV (100 ng) obtained from commensal NL isolates were assessed in the murine BALB/c model. Immune mouse sera were assessed for cross-reactive anti-LOS antibodies by WCE against heat denaturated OMV obtained from meningococcal immunotype reference strains L1 (126E), L3 (6275), L7 (6155), L8 (M978), and L9 (120M) (Table 9). The induction of cross-reactive antibodies after i.v. administration correlated with the expression of immunotype phenotypes (Table 9) and was independent of the meningococcal protein phenotypes. There was no significant differences in the induction of cross-reactive antibodies between whole bacteria, LOS or OMV obtained from NL vaccine isolates.

### Route of administration:

Mucosal administration (oral, nasal, and/or through ear drops), intravenous and/or (sub)cutaneous application are effective ways to induce a lasting immunresponse. Vaccine candidates will be administered directly through solutions, sprays, and/or tablets, and/or through oral administration via vaccine candidates protected with a stomach resistant coating and/or capsule.

**Table 9:**

| Presence of cross-reactive serum antibodies after i.v. administration of whole bacteria, LOS or OMV in BALB/c mice | | | | | |
|---|---|---|---|---|---|
| NL Strain | L1 126E | L3 6275 | L7 6155 | L8 M978 | L9 120M |
| Immunotyp | L1,8 | L(379),8 | L(379),8 | L(379),8,4 | 9,6,8 |
| NL7; Cz4,G03 | + | + | + | ± | + |
| NL1; L01,G01 | ± | + | + | + | + |
| NL13; SR95 | - | + | + | ± | + |
| NL10; SR139 | - | + | + | ± | + |
| NL11; SR319 | + | + | + | + | + |
| NL12; FL671 | ± | + | + | + | + |
| NL3; ICE,G02 | - | + | + | ± | + |
| GRE619 | + | + | + | + | + |
| MC151 | + | + | + | + | ± |
| MC158 | + | + | + | + | ± |
| MC166 | + | + | + | + | + |
| MC179 | + | + | + | + | + |
| MC180 | + | + | + | + | + |
| Data: +, presence of antibodies; -, absence of antibodies; ±, weak induction of functional antibodies compared to the homologous strain | | | | | |

### Discussion of the experimental results

The absorption studies show that antigens found on commensal *Neisseriae* share antigens found on pathogenic meningococci that, surprisingly, are independent on the protein phenotype of commensal bacteria and meningococci. The unexpected variation of phenotypes found in *Neisseria lactamica* strains from different European regions provides evidence of a novel factor that absorbs bactericidal antibodies from normal human adult sera. While endotoxin from *Neisseria meningitidis* is associated with severity and fatality of meningococcal disease, the biological inflammatory activity of endotoxin from commensal *Neisseria lactamica* strains sharing cross-reactive antigens with meningococci show that the commensal LOS molecules are far less toxic compared to meningococcal endotoxin associated with disease. The inflammatory assays showed further, that serum from mice immunised with the commensal *N. lactamica* strains induces functional, meningococcal cross reactive and endotoxin neutralising antibodies. These findings provided evidence, that *N. lactamica* LOS are an effective vaccine inducing protective, antimeningococcal endotoxin antibodies and that antibodies induced by meningococcal and/or commensal *N. lactamica* LOS are an effective treatment in meningococcal induced endotoxic shock.
The experiments measuring the binding of human blood group and meningococcal endotoxin antibodies provide evidence, that cross-reactive antigens were identified as glycoconjugates, namely oligosaccharide antigens found on some strains of *Neisseria lactamica.* Phenotyping of blood group like antigens and meningococcal immunotyping antibodies show further, that meningococcal and commensal *N. lactamica* endotoxin share structural homologous oligosaccharide and core antigens.

### Assessment of the role of M. catarrhalis on the induction of natural immunity to meningococcal disease

Material and Methods were applied as described above for the commensal *Neisseriae* species.

### Results

### Bactericidal assays

The unabsorbed serum pool killed all strains tested (>80 % killing).
**MC1**:In three independent experiments, MC1 absorbed bactericidal activity against MC2 and MC3 but not the other two MC isolates tested. (Table 10). MC1 absorbed bactericidal activity against 13/30 (43%) meningococcal isolates tested: immunotype reference strains L1, L4, L5, and L9 (Table 11); B:15:P1.7,16 from England; B:15:P1.7,16 and C:4:P1.15 from Iceland; B:2a:P1.2, B:15:NT and B:NT:NT from Scotland; B:2a:1.2, B:NT:P1.9 and B:4:P1.15 from Greece (Table 12).
**MC2**: In three independent experiments, MC2 absorbed bactericidal activity against MC1, the Greek NL4 and NL8 from Scotland (Table 10). It absorbed bactericidal activity against 5/30 (17%) meningococcal strains tested: B:2a:P1.2, and B:NT:NT from Scotland; B:2a:P1.2, B:NT:P1.9 and B:4:P1.15 from Greece. All the immunotype reference strains were killed by the sera absorbed with MC2 (Tables 11 and 12).

**Table 10:**

| Absorption of bactericidal activity against MC and NL isolates by MC1 and MC2 (results of 3 independent experiments) | | | |
|---|---|---|---|
| Code | Source | MC1 | MC2 |
| MC1 | Scotland | + | + |
| MC2 | Scotland | + | + |
| MC3 | Scotland | + | - |
| MC4 | Scotland | - | - |
| MC5 | Scotland | - | - |

| | | | |
|---|---|---|---|
| + Reduction in bactericidal activity >800/0 compared with the unabsorbed pool | | | |
| - Reduction in bactericidal activity < 80% compared with the unabsorbed pool | | | |

**Table 11:**

| Absorption of bactericidal activity against meningococcal immunotype reference strains by MC1 or MC2 (results of 3 independent experiments) | | | |
|---|---|---|---|
| Phenotype | LOS oligosaccharide α chain | MC1 | MC2 |
| C:NT:P1.2: **L1** | NeuNAcα (2→3) Galα (1→4) Galβ (1→4) Glcβ | + | - |
| C:2c:P1.1:**L2** | (2→3) Galβ (1→4) GlcNAcβ (1→3) Galβ (1 → 4) Glcβ | - | - |
| B:2a:P1.5,2: **L3** | NeuNAcα (2→3) Galβ (1→4) GlcNAcβ (1→3)-Galβ (1→4) Glcβ | | - |
| C:11:P1.16: **L4** | (2→3) Galβ (1→4) GlcNAcβ (1→3) Galβ (1→ 4) Glcβ | + | - |
| B:4:P1.NT: **L5** | (2→3) Galβ (1→4) GlcNAcβ (1→3) Galβ (1→ 4) Glcβ | + | - |
| B:5:P1.7,1: **L6** | NeuNAcα (2→3) GalNAcβ (1→3) Galα (1→4)-Glcβ | - | - |
| B:9:P1.7,1: **L7** | Galβ (1→4) GlcNAcβ (1→3) Galβ (1→4) Glcβ | - | - |
| B:8,19:P1.7, 1:**L8** | NeuNAcα (2→3) Galβ (1→4) Glcβ | - | - |
| A:21:P1.1.10 :**L9** | Galβ (1→4) GlcNAcβ (1→3) Galβ (1→4) Glcβ | + | - |
| A:21:P1.10: **L10** | Galβ (1→4) GlcNAcβ (1→3) Galβ (1→4) Glcβ | - | - |
| A:21:P1.10: **L11** | Galβ (1→4) Galβ (1→4) Glcβ | - | - |
| A:21:P1.NT: **L12** | | - | - |

| | | | |
|---|---|---|---|
| + Reduction in bactericidal activity ≥80% compared with the unabsorbed pool | | | |
| - Reduction in bactericidal activity < 80% compared with the unabsorbed pool | | | |

Meningococcal immunotypes are highlighted in bold.

**Table 12:**

| Absorption of bactericidal activity against meningococcal isolates from different geographic regions by MC1 or MC2 (results of 3 independent experiments) | | | | |
|---|---|---|---|---|
| Phenotype | No. | Origin | MC1 | MC2 |
| B:15:P1.7,16 | A11 | England | + | - |
| B:NT:P1.9 | 1766 | Greece | + | + |
| B:NT:P1.13 | PE255 | Greece | - | - |
| NG:NT:NT | ST776 | Greece | - | - |
| NG: NT: NT | P481 | Greece | - | - |
| B:2a:P1.2 | TH39 | Greece | - | - |
| B:2a:P1.2 | TH44 | Greece | + | + |
| B:4:P1.15 | A43 | Greece | + | + |
| C:2a:P1.2 | A14 | Greece | - | - |
| C:4:NT | A26 | Greece | - | - |
| NG:4:NT | A48 | Greece | - | - |
| B:15:P1.7,16 | B14 | Iceland | + | - |
| C:4:P1.15 | Ice155 | Iceland | + | - |
| B:15:NT | 99-1787 | Scotland | + | - |
| B:2a:P1.2,5 | Sto B | Scotland | - | - |
| B:NT:NT | 99/760 | Scotland | + | + |
| C:2a:NT | A25 | Scotland | - | |
| C:2a:P1.2 | StoC | Scotland | - | - |
| B 2a:P1.2 | SNMP | Scotland | + | + |

| | | | | |
|---|---|---|---|---|
| + Reduction in bactericidal activity ≽:80% compared with the unabsorbed pool | | | | |
| - Reduction in bactericidal activity < 80% compared with the unabsorbed pool | | | | |

### Assessment of binding of blood grouping and meningococcal immunotypinq antibodies

Clinical isolates of MC (n=126) were from our culture collection. The binding of blood group antibodies against P, P1, p^{K}, paragloboside, I and meningococcal immunotype L(3,7,9) were measured by WCE. Because of the large number of strains to be tested and the limited amount of reagents, WCE was used and only the L(3,7,9) monoclonal tested as this epitope is most likely to be the one involved in induction of protective antibodies against disease causing strains.

MC1 bound antibodies to P, P1, p^{K} and I; MC2 bound only antibodies to p^{K}, None of the two strains bound antibodies to paragloboside or L(3,7,9).

Most clinical isolates of MC bound one or more antibody to the following antigens (Table 13): P (12.7%); P1 (23.8%); p^{K} (63.5%); paragloboside (17.5%); I (19.0%); and L(3,7,9) (30.2%).

**Table 13:**

| WCE for binding of antibodies to blood group antigens and L(3,7,9) by *M. catarrhalis* strains | |
|---|---|
| Antigen | Positive MC strains n=126, No (%) |
| P | 16 (12.7) |
| P1 | 30 (23.8) |
| p^{K} | 80 (63.5) |
| Paragloboside | 22 (17.5) |
| I | 24 (19.0) |
| No binding of blood group antibodies tested | 33 (26.2) |
| L(3,7,9) | 38 (30.2) |

### Binding of antibodies to blood group antigens by M. catarrhalis isolates from Scotland

The binding of blood group antibodies against P, P1, pK, paragloboside (n=126) and meningococcal immunotype L(3,7,9) (n=187) to clinical isolates of *M*. *catarrhalis* from our bacteria collection obtained from adults (n=100) and children (n=26 for blood group antibodies, or n=87 for meningococcal immunotype antibodies). Binding of antibodies to pK did not differ significantly between isolates obtained from adults or children, while a greater number of isolates obtained from children bound other blood group antibodies tested. (Table 14).

**Table 14:**

| Binding of antibodies to blood group antigens antibodies by *M*. *catarrhalis* strains obtained from adults (n=100) and children (n=26) | | | | | | |
|---|---|---|---|---|---|---|
| Antigen | Strains from (n=100) | isolated adults | Strains from (n=26) | isolated children | Total (n=126) | strains |
| P | 15 (15) | | 1 (3.8) | | 16 (12.7) | |
| P1 | 28 (28) | | 2 (7.7) | | 30 (23.8) | |
| pK | 61 (61) | | 19 (73.1) | | 80 (63.5) | |
| Paragloboside | 21 (21) | | 1 (3.8) | | 22 (17.5) | |
| I | 23 (23) | | 1 (3.8) | | 24 (19.0) | |
| No binding of blood group antibodies | 25 (25) | | 6 (23.1) | | 33 (26.2) | |
| Data: positive cells (%age of positive cells) | | | | | | |

### Binding of antibodies to meningococcal immunotype antigens by M. catarrhalis isolates from Scotland

Significantly more strains isolated from children (n=87) bound antibodies to the meningococcal immunotype L(3,7,9) associated to disease to isolates obtained from adults (n=100). Most isolates obtained from children (74.77%) bound antibodies to meningococcal immunotype L1, and only few (5.7%) bound immunotype L8 antibodies (Table 15).

**Table 15:**

| Binding of antibodies to blood group antigens and meningococcal immunotype antibodies by *M*. *catarrhalis* strains obtained from adults (n=100) and children (n=26) | | |
|---|---|---|
| Antigen | Strains isolated from adults (n=100) | Strains isolated from children(n=87) |
| L1 | n.t. | 65 (74.7) |
| L(3,7,9) | 23 (23) | 32 (36.8) |
| L8 | n.t. | 5 (5.7) |
| Data: positive cells (%age of positive cells); n.t.: not tested | | |

### Inflammatory response of LOS from meningococci and MC isolates

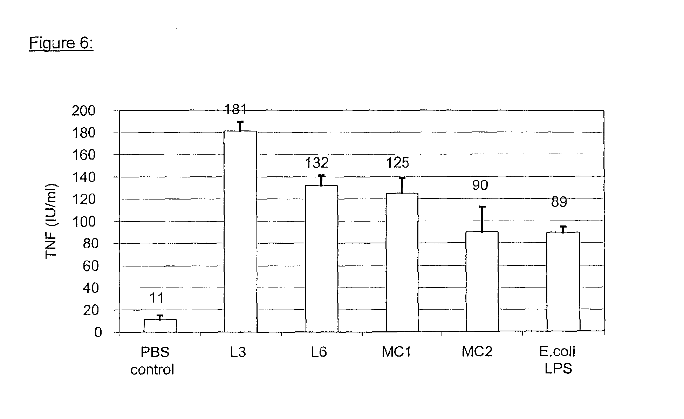

In six independent experiments in which each control and test condition was carried out in triplicate, VD3 differentiated THP-1 cells were challenged with LOS (100 pg ml⁻¹) from meningococcal immunotypes L3, L6, from MC1, MC2 and *E. coli* endotoxin. LOS from the L3 immunotype induced significantly higher TNFα levels compared to TNFα levels obtained with LOS from MC1, MC2 and *E.coli* (**P<0.01**) (Figure 6). MC2 induced significantly lower levels of TNFα compared to immunotype L3, L6 and MC1 but not *E. coli* LPS (**P<0.05**).

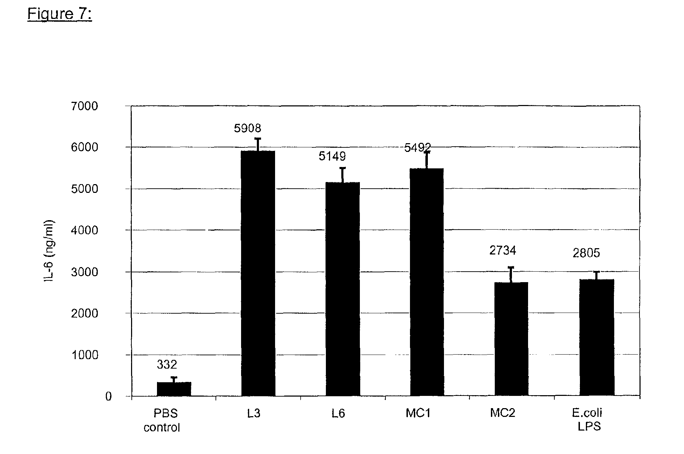

In six independent experiments, a similar pattern was observed for induction of IL-6. All except LOS from MC2 induced significantly higher levels of IL-6 compared with the *E. coli* LPS (Figures 7). MC2 LOS preparations and *E. coli* LPS elicited IL-6 levels significantly lower than those obtained with LOS from meningococcal immunotype strains L3, L6, and MC1 **(P<0.01)**.

### Discussion

The absorption studies show that antigens found on *M. catarrhalis* strains share, and induce functional and bactericidal antibodies against antigens found on pathogenic meningococci. Similar to commensal *Neisseriae* strains, endotoxin obtained from some *M. catarrhalis* strains induces significantly lower cytokine levels compared to meningococcal endotoxin immunotype L(3,7,9).

The endotoxin moieties were identified to share structural and antigenetic homology with human blood group and meningococcal endotoxin antigens, and are, similar to commensal *Neisseriae,* the main molecule with cross-reactive antigenicity to meningococcal endotoxins.

*M. catarrhalis* isolates are commonly found as commensal strains in the nasopharynx of young children, as well are a common infectious agent for childhood otitis media. The experiments presented provide evidence that the carriage of, or infection with *M*. *catarrhalis* induces functional antibodies directed against meningococcal endotoxin, and that endotoxin obtained from *M. catarrhalis* is a vaccine for the protection against meningococcal disease.

### Explanation of the Figures:

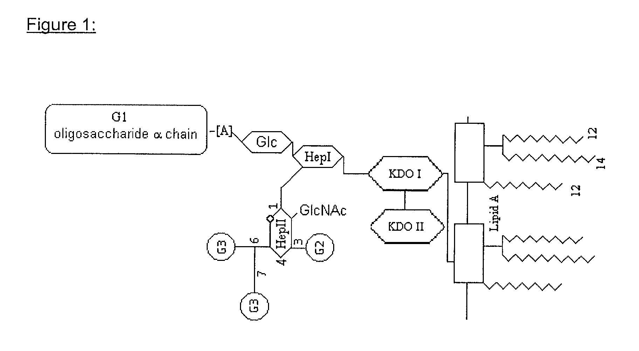

Figure 1: Schematic structure of meningococcal LOS immunotypes G3: PEA (1 →6) L2, L4, L6; G2: PEA (1→3) L1, L8, L(3,7,9); H (→3) L4, L6; αGal (1→3) L2
Figure 2: TNFα (IU ml⁻¹) responses to LOS from meningococci (L3, L6), *N. lactamica* 1 or *E.coli* endotoxin (100 pg ml⁻¹) by (a) undifferentiated, (b) differentiated THP-1 cells (n=6, error bars = SD)
Figure 3: IL-6 (pg ml⁻¹) responses to LOS of meningococci (L3, L6), commensal isolates *N. lactamica* 1 or *E. coli* endotoxin (100 pg ml⁻¹) by (a) undifferentiated, (b) differentiated THP-1 cells (n=6, error bars = SD)
Figure 4: (a) Release of TNFα (IU ml⁻¹) from VD3 differentiated THP-1 cells challenged with meningococcal LOS, *N. lactamica* 1 LOS or *E. coli* LPS (100 pg ml⁻¹), (b) endotoxins co-incubated with pooled human serum (final dilution 1 in 1000), (c) endotoxins co-incubated with immune mouse serum produced by vaccination with *N. lactamica* 1 (final dilution 1 in 1000) (n=6; error bars = standard deviation)
Figure 5: (a) Release of IL-6 (pg ml"¹) from VD3 differentiated THP-1 cells challenged with meningococcal LOS, *N. lactamica* 1 LOS or *E. coli* LPS (100 pg ml⁻¹), (b) endotoxins co-incubated with pooled human serum (final dilution 1 in 1000), (c) endotoxins co-incubated with immune mouse serum produced by vaccination with *N. lactamica* 1 (final dilution 1 in 1000) (n=6; error bars = standard deviation)
Figure 6: TNFα (IU ml⁻¹) responses to LOS from meningococci (L3, L6), commensal species (NL1, MC1, MC2) or *E.coli* endotoxin (100 pg ml⁻¹) by differentiated THP-1 cells (n=6, error bars = SD)
Figure 7: IL-6 (ng ml⁻¹) responses to LOS of meningococci (L3, L6), commensal isolates (NL1, MC1/ MC2) or *E. coll* endotoxin (100 pg ml⁻¹) by differentiated THP-1 cells (n=6, error bars = SD)
Figure 8: Capsular polysaccharide, outer membrane proteins (OMP) and transmembrane proteins, pili, lipooligosaccharide (LOS) of meningococci.

## Claims

1. A medicament for the treatment or prevention of diseases due to infection by *Neisseria meningitidis,* **characterized in that** it comprises glycoconjugates and/or lipooligosaccharides (LOS) from commensal bacteria with cross-reactive antigens to *Neisseria meningitidis* and/or antibodies against such glycoconjugates and/or lipooligosaccharides.

2. The medicament of claim 1, **characterized in that** the commensal bacteria with cross-reactive antigens to *Neisseria meningitidis* are selected from the group consisting of *Moraxella catarrhalis* and/or *Neisseria lactamica.*

3. The medicament of claim 1, **characterized in that** the *Neisseria meningitidis* are of the serogroup A, B, C, H, I, K, L, X, Y, Z, 29E or W135, or non-capsulated meningococcal strains.

4. The medicament of claim 1, **characterized in that** the glycoconjugates and/or lipooligosaccharides are included in outer membrane vesicles, blebs, lipid layers, liposomes and/or killed or viable bacteria commensal to *Neisseria meningitidis.*

5. The medicament of claim 1, **characterized in that** the glycoconjugates and/or lipooligosaccharides are chemically modified, conjugated and/or hydrolyzed, preferably by mild acid hydrolysis.

6. The medicament of claim 1, preferably for the treatment of acute meningitis or septicaemia, **characterized in that** the antibodies are monoclonal or polyclonal, and that they are obtained from commensal and/or meningococcal species from:
- virus immortalized human lymphocytes secreting the glycoconjugate neutralizing, specific or cross-reactive antibodies,
- from human lymphocytes secreting the neutralizing antibodies fused with a human hybridoma cell line,
- from immunized animals, preferably mice, rats, rabbits or pigs producing polyclonal serum against such antibodies, or
- from immunized animals, preferably mice, rats, rabbits or pigs, after fusion of the mouse lymphocytes with a human or animal hybridoma cell line.

7. The medicament of claim 1, **characterized in that** it is a vaccine.

8. The medicament of claim 1, **characterized in that** it is provided as a nasal/oral spray, as a liquid for injection, as an orally applied capsule or tablet and/or in combination with an adjuvant.

9. A diagnostic to assess the susceptibility of patients for diseases due to *Neisseria meningitidis,* **characterized in that** it comprises glycoconjugates and/or lipooligosaccharides from commensal bacteria with cross-reactive antigens to *Neisseria meningitidis* and/or antibodies against such glycoconjugates and/or lipooligosaccharides of any of the claims 1 to 8.

10. The use of glycoconjugates and/or lipooligosaccharides (LOS) from commensal bacteria with cross-reactive antigens to *Neisseria meningitidis* and/or antibodies against such glycoconjugates and/or lipooligosaccharides for the preparation of a medicament for the treatment of diseases due to infection by *Neisseria meningitidis.*
